Europäisches Patentamt

European Patent Office

Office européen des brevets

(11) Veröffentlichungsnummer: **0 448 004 A2**

## (12) EUROPÄISCHE PATENTANMELDUNG

(21) Anmeldenummer: **91104121.8**

(22) Anmeldetag: **15.03.91**

(51) Int. Cl.5: **A61B 17/22**

(30) Priorität: **19.03.90 DE 9003184 U**

(43) Veröffentlichungstag der Anmeldung:
**25.09.91 Patentblatt 91/39**

(84) Benannte Vertragsstaaten:
**CH DE DK FR GB IT LI SE**

(71) Anmelder: **Weikl, Andreas, Dr.**
**Kosbacher Weg 51b**
**W-8520 Erlangen(DE)**

Anmelder: **Merkel, Volkmar, Dipl.-Ing.(FH)**
**Willi-Grasser-Strasse 9**
**W-8520 Erlangen(DE)**

(72) Erfinder: **Weikl, Andreas, Dr.**
**Kosbacher Weg 51b**
**W-8520 Erlangen(DE)**
Erfinder: **Merkel, Volkmar, Dipl.-Ing.(FH)**
**Willi-Grasser-Strasse 9**
**W-8520 Erlangen(DE)**

(74) Vertreter: **Hufnagel, Walter, Dipl.-Ing.,**
**Dipl.-Wirtsch.-Ing. et al**
**Dorner & Hufnagel Patentanwälte Bad**
**Brückenauer Str. 19**
**W-8500 Nürnberg 90(DE)**

(54) **Behandlungskatheter.**

(57) Ein Behandlungskatheter zur Verringerung oder Beseitigung von Engstellen in Körperflüssigkeit führenden Gefäßen, mit wenigstens einem über ein Lumen oder über einen Kanal durch ein flüssiges Medium aufweitbaren Ballon, sowie mit einer Lichtleitfaser, durch die ein von außen in dieses bzw. in diesen eingeleitetes Laserlicht beim im Gefäß entsprechend positioniertem Katheter im Bereich der Engstelle abstrahlbar ist soll so ausgebildet werden, daß im Bereich der Verengungsstelle eine möglichst gleichmäßige, ausreichende Erhitzung des Gewebes erreichbar ist. Dies wird nach einer Variante der Erfindung dadurch erreicht, daß wenigstens eine Lichtleitfaser (11) im Bereich (5) innerhalb eines Ballons (4) derart ausgebildet ist, daß die einstrahlbare Laserenergie in den Ballon (4) abstrahlbar ist und daß der Ballon (4) mit einem Fluid füllbar und aufweitbar ist, das ein hohes Absorptionsvermögen für die Wellenlänge des verwendeten Laserlichts besitzt.

Fig. 1

EP 0 448 004 A2

Die vorliegende Erfindung bezieht sich auf einen Behandlungskatheter zur Verringerung oder Beseitigung von Engstellen in Körperflüssigkeit führenden Gefäßen gemäß dem Oberbegriff des Anspruches 1.

Ein derartiger Behandlungskatheter ist aus dem Aufsatz "Laser Balloon Angioplasty: An Optical and Thermal Analysis" von A.J.Welch und Wai-Fung Cheong aus dem Biomedical Engineering Programm, The University of Texas at Austin, Austin, Texas 78712, Seiten 68 bis 95 bekannt. Mit derartigen Kathetern werden Stenosen in Blutgefäßen aufgeweitet, das Gewebe durch Erhitzen denaturiert und damit die Aufweitung stabilisiert. Es hat sich nämlich gezeigt, daß bei Aufweitungen ohne die erwähnte Thermostabilisierung Proliferationen auftreten, die nach gewisser Zeit eine erneute Behandlung notwendig machen können. Bei diesem bekannten Katheter, der im vorgesehenen Behandlungsbereich einen durch ein Fluid aufdehnbaren Ballon aufweist, ist eine Lichtleitfaser zentral angeordnet, die im Bereich des Ballons ein von außen in die Lichtleitfaser eingetrahltes Laserlicht, beispielsweise eines $CO_2$-Lasers oder eines Nd:YAG-Lasers (Neodym-Yttrium-Aluminium-Granat-Laser), diffus abstrahlt. Das abgestrahlte Laserlicht durchdringt die Ballonwand und trifft bei aufgedehntem Ballon von innen auf die Oberfläche des zu behandelnden, insbesondere zu dilatierenden verengten Gewebes, beispielsweise in Arterien. Die Aufdehnung des Ballons erfolgt durch ein das zur Anwendung gelangende Laserlicht praktisch nicht absorbierendes und vorzugsweise nicht streuendes Fluid. Durch die Bestrahlung mit Laserlicht wird die Oberfläche des Gewebes der Verengungsstelle relativ schnell auf eine hohe Temperatur von 200°C oder auch mehr erhitzt und die Wärme in das Gewebeinnere durch die Wärmeleitung des wenig wärmeleitfähigen Gewebes nach innen verteilt. Hierdurch kann die Gefahr der Entstehung von Proliferationen zwar verringert und es können Restenosierungen u.U. auch weitestgehend verhindert werden. Es besteht jedoch die Gefahr, daß die Oberfläche des Gewebes an der Verengungsstelle zu stark denaturiert bzw. teilweise durch Verkohlung oder Schorfbildung sogar zerstört wird. Um diese Gefahr zu reduzieren, erfolgt die Energiezufuhr zeitabhängig mit abnehmender Energiedichte. Andererseits ist aber der Grad der Erhitzung der Oberfläche des Gewebes der Engstelle vom Absorptionsvermögen dieser Oberfläche abhängig. Insbesondere bei krankhaften Gewebeveränderungen ist das Absorptionsvermögen örtlich zum Teil stark unterschiedlich ausgebildet, so daß unter Umständen das zu behandelnde Gewebe der Engstelle weniger stark erhitzt wird als das nicht behandlungsbedürftige gesunde Gewebe. Das unterschiedliche Absorptionsvermögen des zu behandelnden Gewebes der Verengungsstelle ist in der Regel aber nicht vorherbestimmbar. Die örtliche Entstehung von Proliferationen und/oder übermäßiger Denaturierungen kann daher mit dem vorbekannten Behandlungskatheter nicht ausgeschlossen werden.

Mit der vorliegenden Erfindung soll demgegenüber die Aufgabe gelöst werden, einen Behandlungskatheter der eingangs erwähnten Art so weiterzubilden, daß im Bereich der Verengungsstelle eine möglichst gleichmäßige, ausreichende Erhitzung des Gewebes erreichbar ist, insbesondere aber eine partiell zu hohe Erhitzung der inneren Gewebeoberfläche vermieden wird . Insbesondere werden auch Bestrahlungsschäden vermieden, wie sie bei der bekannten direkten Einstrahlung von Laserlicht vielfach auftreten.

Gelöst wird diese Aufgabe durch die im Kennzeichen des Anspruches 1 angegebenen Merkmale.

Mit der vorliegenden Erfindung wird gemäß dem Merkmal a) des Kennzeichens des Anspruchs 1 das zum Aufdehnen des Ballons dienende Fluid direkt durch Laserenergie aufgeheizt. Dadurch wird eine gleichmäßige Erhitzung des Gewebes der Engstelle über die gesamte Ballonaußenfläche gewährleistet, ohne daß es zu partiellen Überhitzungen aufgrund von unterschiedlichen Laserlicht-Absorptionsgraden kommen kann. Außerdem muß bei der Auswahl des Materials für den Ballon nicht darauf geachtet werden, welches Absorptionsvermögen und/oder Streuvermögen dieser bezüglich der Wellenlänge des verwendeten Laserlichts besitzt. Damit ist auch eine Optimierung in der Zuordnung der Materialien für den eigentlichen Behandlungskatheter einerseits und den Katheterballon andererseits möglich.

Gemäß dem Merkmal b) des Kennzeichens des Anspruchs 1 wird das Fluid im Bereich zwischen zwei ausdehnbaren Ballons durch die Laserenergie aufgeheizt und die Wärme unmittelbar an das Gewebe übertragen. Auch hierbei wird eine gleichmäßige Erhitzung ohne die Gefahr partieller Überhitzung von Gewebestellen ermöglicht. Hierdurch kann eine Stabilisierung des erhitzten Gewebes erreicht werden. Zugleich besteht hierbei die Möglichkeit, die Erhitzung so durchzuführen, daß das Gewebe an der Oberfläche destabilisiert oder denaturiert wird, unter der Oberfläche jedoch eine Stabilisation auftritt. Bei Anwendung des Doppelballonkatheters können dann in den Bereich zwischen den beiden Ballons ein Lösungs- und Durchspülmittel eingebracht und eine chemische, enzymatische oder angiotropische Behandlung durchgeführt und die destabilisierten oder denaturierten Gewebestrukturen aufgelöst und durch Spülen entfernt werden.

Weitere vorteilhafte Eigenschaften der Erfindung sind in den Unteransprüchen angegeben und werden nachfolgend anhand der in der Zeichnung veranschaulichten Ausführungsbeispielen näher beschrieben:

Es zeigen:

Figuren 1 und 2
je einen in ein Blutgefäß mit einer Stenose eingeführten Katheterabschnitt von der Seite im Schnitt gesehen, mit im Lumen des Behandlungskatheters vorgesehener Lichtleitfaser,

Figur 3
einen Behandlungskatheterabschnitt von der Seite im Schnitt, wobei die Lichtleitfaser an der Außenfläche des Behandlungskatheters angebracht und eine axiale Einstrahlung des Laserlichts vorgesehen ist,

Figur 4
einen Behandlungskatheter von der Stirnseite gesehen als Prinzipskizze,

Figuren 5 und 6
je einen in ein Blutgefäß mit einer Stenose eingeführten Katheterabschnitt von der Seite im Schnitt mit axialer Einstrahlung des Laserlichts in ein Lichtverteilerelement,

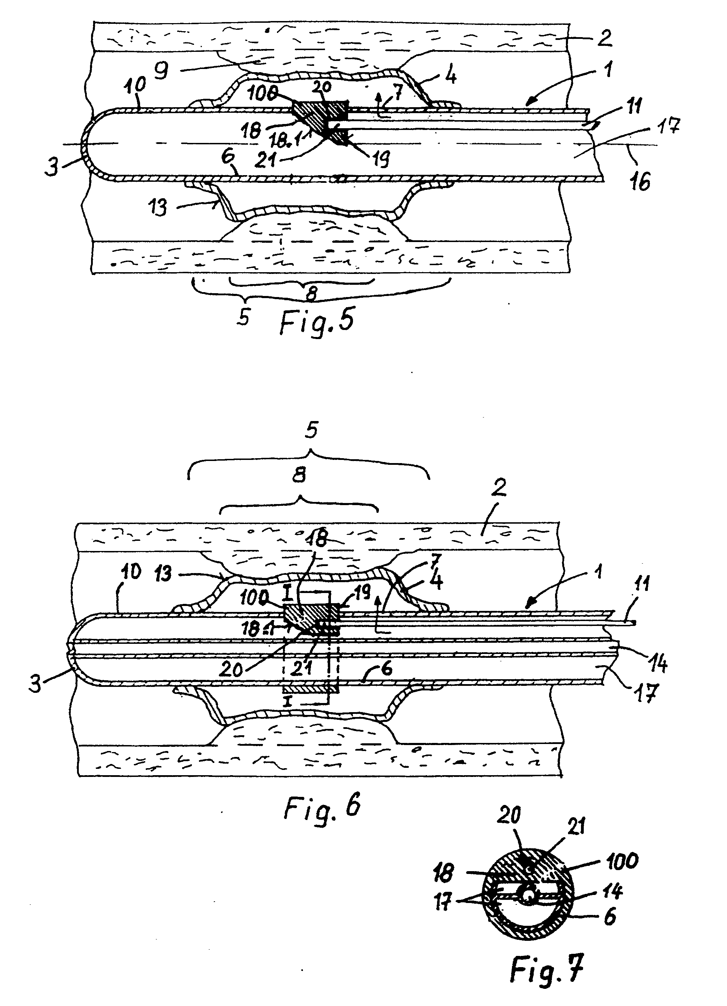

Figur 7
eine Ansicht gemäß der Schnittlinie I-I der Figur 6,

Figur 8
den Endabschnitt eines Doppelballonkatheters mit im Lumen verlaufenden Lichtleiter von der Seite im Schnitt gesehen,

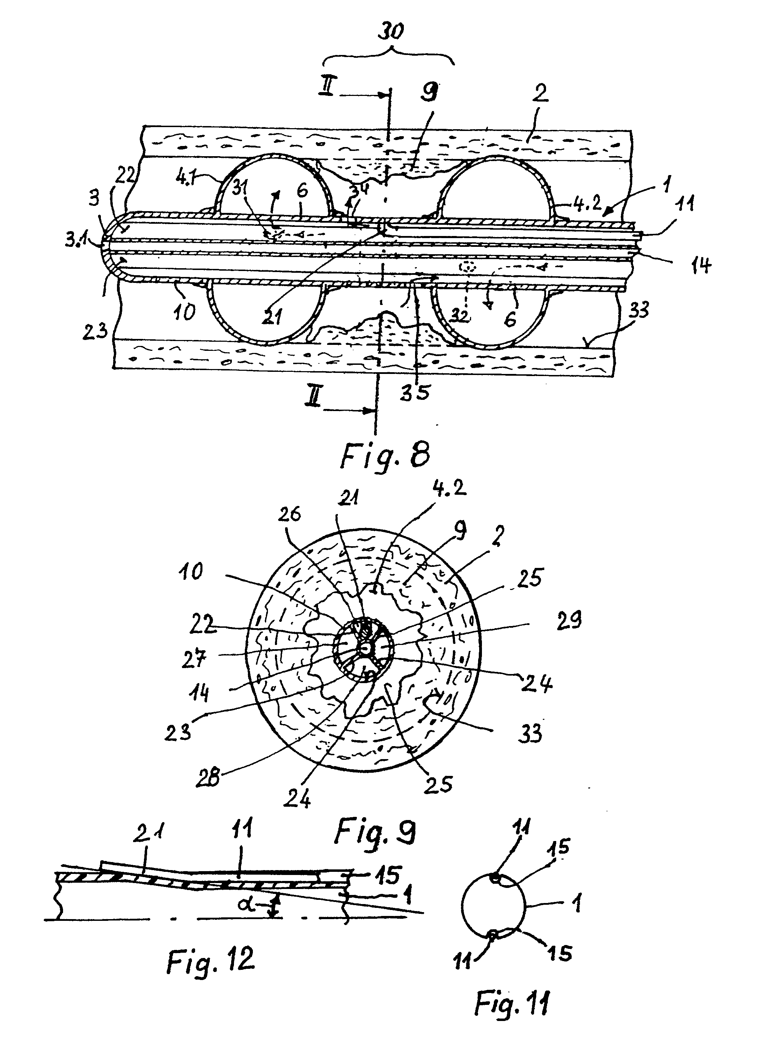

Fig. 9
einen Querschnitt durch den Doppelballonkatheter der Figur 8 gemäß der Schnittlinie II-II,

Fig. 10
einen Abschnitt eines Doppelballonkatheters mit an der Außenfläche des Behandlungskatheters verlaufenden Lichtleiter von der Seite im Schnitt gesehen, mit axialer Einstrahlung des Laserlichts,

Fig. 11
einen Querschnitt durch einen Doppelballonkatheter im Bereich zwischen den Ballons mit zwei vertieft außen am Lumen verlaufenden Lichtleitern,

Fig. 12
einen Ausschnitt eines Katheters mit außem am Lumen vertieft verlaufenden Lichtleiter und schräg nach außen abstehendem Lichtleiterende,

Fig. 13 und 14
je einen Doppelballonkatheterabschnitt von der Seite im Schnitt gesehen mit je einem Lichtverteilelement,

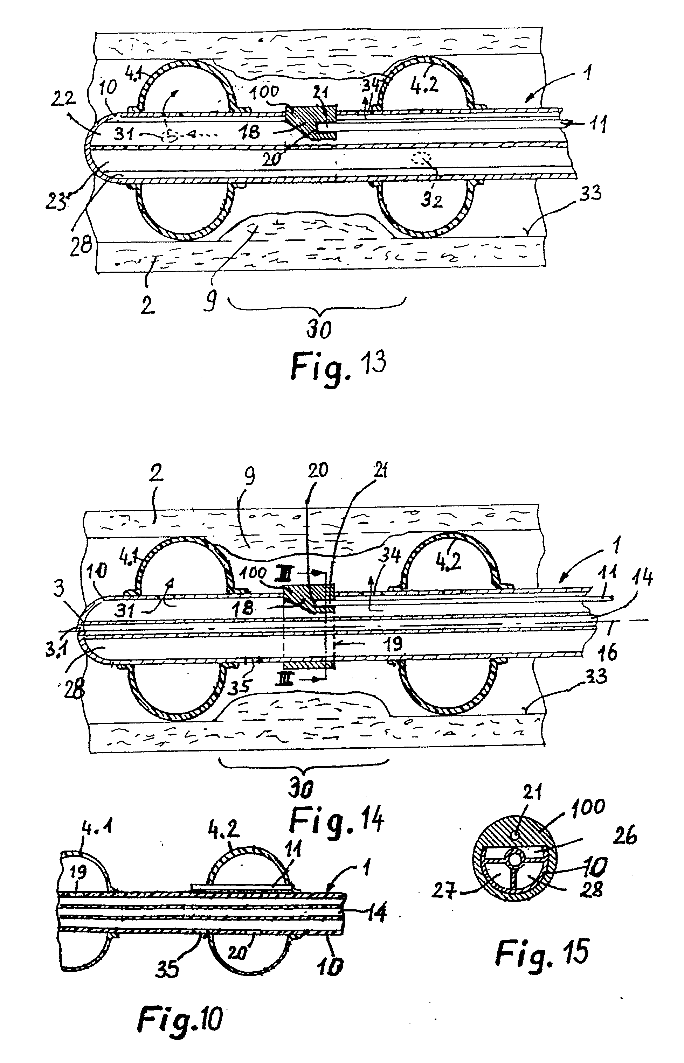

Fig. 15
einen Schnitt gemäß den Linien III-III der Figur 14,

Fig. 16 bis 18

Ausschnitte von Ausführungsmöglichkeiten einer Ballonwand im Schnitt,

Fig. 19 und 20
je einen Ausschnitt einer mit Partikeln versetzten Ballonwand im Schnitt,

Fig. 21 bis 23
mögliche Formen von Lichtverteilelementen,

Fig. 24
eine spiralförmige Ausbildung eines Lichtleiterendes und

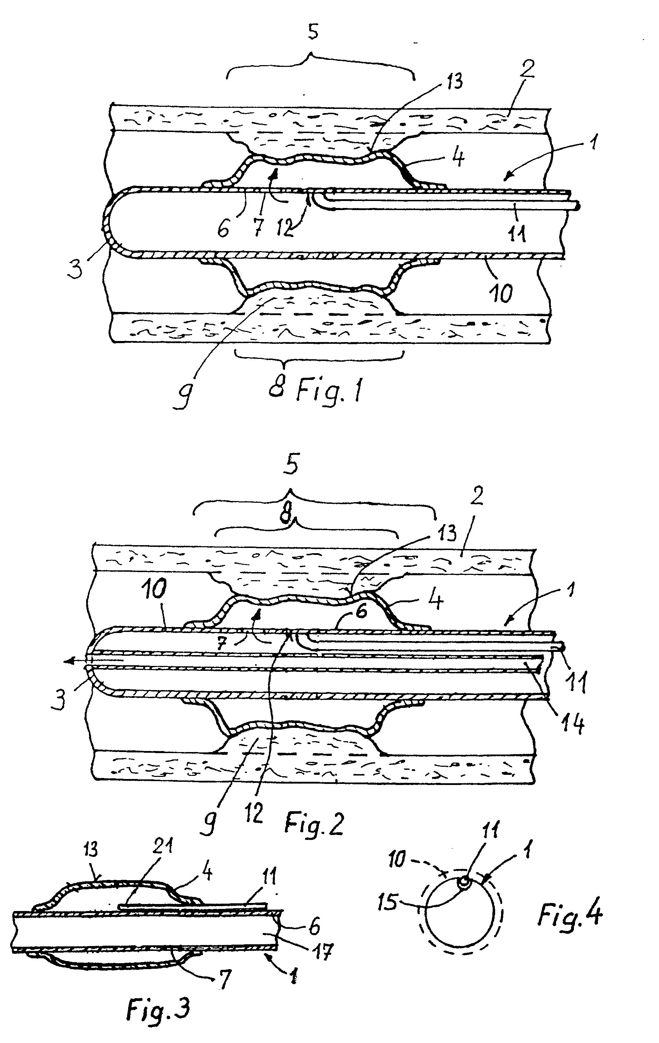

Fig. 25
eine weitere Ausführungsform eines Behandlungskatheters gemäß Fig. 2 mit vergrößerter Absorptionsstrecke A.

In Figur 1 ist mit 1 ein Behandlungskatheter bezeichnet, der im Bereich des in ein Blutgefäß 2, beispielsweise einer Arterie, eingeführten abgeschlossenen Endes 3 mit einem ausdehnbaren Ballon 4 versehen ist, der sich über einen Abschnitt 5 des Endes des Behandlungskatheters 1 erstreckt. Innerhalb des Ballons 4 ist die Wand 6 des Behandlungskatheters 1 mit einer Öffnung 7 versehen. Durch diese Öffnung 7 kann von außen ein flüssiges Medium, beispielsweise Wasser, eine Kochsalzlösung oder dgl., unter Druck in den Innenraum des Ballons 4 gedrückt und der Ballon 4 dadurch aufgedehnt werden. Dies erfolgt in einem Behandlungsbereich 8 an einer Stelle des Blutgefäßes 2 oder eines anderen Gefäßes, wo dieses eine zu beseitigende Engstelle 9 aufweist.

Im Lumen oder auch im Bereich der Außenwand 10 des Katheters 1 ist eine Lichtleitfaser 11 vorgesehen, in die von außen Laserlicht einstrahlbar ist. Die Lichtleitfaser 11 ist derart ausgebildet und angeordnet, daß in die Lichtleitfaser 11 von außen eingestrahltes Laserlicht gegen das im Ballon 4 befindliche Fluid, beispielsweise in Richtung der inneren Oberfläche 12 des Ballons 4 abgestrahlt wird. Damit eine Erhitzung des Fluids durch das Laserlicht mit hohem Wirkungsgrad möglich ist, besteht dieses aus einem Medium oder enthält fein verteilt ein solches Medium, das für die Wellenlänge des verwendeten Laserlichts ein hohes Absorptionsvermögen besitzt. Geeignet sind beispielsweise feinst dispergierte Farbpigmente oder Metalle, wie Eisen, Kupfer, Edelstahl, Titan oder dgl. oder auch gut wärmeleitende andere Werkstoffe, wie Metallkarbide, Metallnitride oder keramische Zusammensetzungen, beispielsweise Aluminiumoxid oder auf der Basis von Aluminiumoxid oder dgl. gebildete Zusammensetzungen. Diese Pigmente oder dgl. können einer Kochsalzlösung oder Elektrolytlösung als Trägerflüssigkeit zugesetzt sein. Es können jedoch auch farbige, pigmentfreie Fluids Verwendung finden.

Das Laserlicht kann direkt oder über ein Lichtverteilelement gegen das Fluid abgestrahlt werden. Hierbei muß auf das Absorptionsvermögen des Laserlichtes durch die Wand 6 des Behandlungskatheters 1 keine Rücksicht genommen werden. Tritt das Laserlicht jedoch durch die Wand 6 des Behandlungskatheters 1 hindurch, wie beim Ausführungsbeispiel gemäß Figur 1 dargestellt, dann wird als Material für den Behandlungskatheter 1 zumindest im betreffenden Abschnitt ein solches verwendet, das das eingestrahlte Laserlicht nicht oder so gut wie nicht absorbiert.

Vorteilhaft erfolgt die Einstrahlung des Laserlichts etwa in der Mitte oder wenigstens annähernd in der Mitte des Ballonabschnittes 5, um eine möglichst gleichmäßige Aufheizung des flüssigen, zur Aufdehnung des Ballons 4 verwendeten Mediums zu erreichen.

Die Anwendung des erfindungsgemäßen Behandlungskatheters 1 erfolgt beispielsweise derart, daß dieser zunächst in das Gefäß 2, vorzugsweise in ein Blutgefäß, eingeführt und der Abschnitt 5 des Behandlungskatheters 1 an die Engstelle 9 gebracht wird. Anschließend wird der Ballon 4 aufgedehnt, indem das das verwendete Laserlicht absorbierende Fluid unter Druck gesetzt wird. Dabei drückt sich die Ballonaußenwand 13 gegen das Gewebe der Engstelle 9, vorzugsweise gegen eine Stenose. Gleichzeitig oder unmittelbar nach Beginn der Ausdehnungsphase wird Laserlicht vorbestimmter Leistung, Wellenlänge und Einwirkungsdauer in die Lichtleitfaser 11 eingestrahlt und damit das im Ballon 4 befindliche Fluid erhitzt. Die Temperatur des Fluids wird über die Ballonhülle des Ballons 4 in das Gewebe der Engstelle 9 geleitet. Das erwärmte oder erhitzte Gewebe wird an der Engstelle 9 durch Aufblähen des Ballons 4 aufgeweitet. Durch die über den Umfang gleichmäßig erhöhte Gewebetemperatur kommt es dabei nicht zu Rissen in der Gewebestruktur der Verengungsstelle, sondern vielmehr im aufgeweiteten Zustand des Ballons 4 zu einer gleichmäßig stabilisierenden Veränderung der Oberfläche des Gewebes, insbesondere zu einer Verschweißung der Gewebeoberfläche mit der Folge, daß Proliferationsprozesse weitestgehend ausgeschlossen sind.

Unter dem Begriff "Denaturierung" des Engstellenmaterials ist eine Umstrukturierung von dessen Eiweißmolekülen aufgrund der vorgenommenen Wärmebehandlung zu verstehen.

Gemäß der in Figur 2 dargestellten, vorteilhaften Weiterbildung der Erfindung kann der Behandlungskatheter 1 zusätzlich mit einem vorzugsweise zentralen Versorgungskanal 14 versehen sein. Durch diesen kann das Gefäß, insbesondere Blutgefäß, mit während der Behandlungsphase notwendigen Stoffen, beispielsweise mit Blut, Blutersatz, Kochsalzlösungen, Medikamenten oder dgl. auch bei aufgedehntem Ballon 4 versorgt bzw. weiterversorgt werden.

Unter Umständen kann es auch zweckmäßig sein, wenigstens eine Lichtleitfaser 11, ggf. auch zusätzlich, außen am Katheter 1, (Figur 3) oder insbesondere in einer Längsnut 15 des Katheters 1 (Figur 4), anzubringen und über diese Lichtleitfaser 11 das Fluid zu erhitzen.

Gemäß einer vorteilhaften weiteren Ausgestaltung der Erfindung können die Lichtleitfasern 11 oder es kann wenigstens eine der Lichtleitfasern 11 derart ausgebildet und angeordnet sein, daß die Lichtleitfaser(n) 11 axial, d.h. in Richtung der Längsachse 16 (Figur 5) des Behandlungskatheters 1 in ein Lichtverteilelement 100 einstrahlt bzw. einstrahlen. Hierzu ist gemäß den Figuren 5 bis 7 der das Lichtverteilelement 100 mit einen in das Lumen 17 oder in einen Kanal hineinragenden Abschnitt 18 versehen. Dieser Abschnitt 18 besitzt auf der der Lichtleitfaser 11 zugewandten Stirnseite 19 eine Aussparung 20 in Form eines axial, d.h. in Richtung der Längsachse 16, verlaufenden Sackloches. In dieses ist das abstrahlende Ende 21 der Lichtleitfaser 11 eingeführt. Das Ende 21 der Lichtleitfaser 11 kann in der Aussparung 20 beispielsweise durch Klebung befestigt sein. Je Aussparung 20 können mehrere Lichtleitfasern 11 vorgesehen sein. Auch können mehrere Aussparungen 20 vorhanden sein, in die jeweils wenigstens eine Lichtleitfaser 11 hineinragt. Das Lichtverteilelement 100 besitzt am Abschnitt 18 eine im Strahlengang des Laserlichts liegende Prismenfläche 18.1, die das Laserlicht nach außen umlenkt. Ein Lichtverteilelement 100 kann auch bei außen am Katheter 1 verlaufender Lichtleitfaser angewendet werden. Zweckmäßig ist dessen abstrahlende Oberfläche lichtstreuend ausgebildet. Durch ein Lichtverteilelement 100 kann die Energiedichte der Laserstrahlung gegenüber derjenigen in der Lichtleitfaser stark reduziert werden, so daß es auch bei sehr hoher Energiedichte nicht zum Verdampfen von Fluidbestandteilen kommt.

Bei allen Ausführungsformen kann das Lichtverteilelement 100 an seiner Oberfläche mit Rippen oder mit anderen, dessen Oberfläche vergrößernden Mitteln versehen sein.

Der erfindungsgemäße Behandlungskatheter 1 für die Behandlung von Engstellen kann bei allen Arten von Körperflüssigkeit führenden Gefäßen, also bei allen Arten von Blutgefäßen oder andere Flüssigkeiten führenden Gefäßen, wie Liquorkanälen oder auch bei größeren Lymphgefäßen eingesetzt werden. Insbesondere kommt auch eine Aufweitung von Engstellen in Harnleitern in Betracht.

Als weitere Anwendungsgebiete kommen beispielsweise auch Behandlungen von Verengungen im Gallenbereich, im Pankreas-Ausführungsgang oder im Rückenmarkskanal oder dgl. in Betracht.

In den Figuren 8 und 9 ist mit 1 ein Behandlungskatheter bezeichnet, der beispielsweise einen vorzugsweise zentralen Versorgungskanal 14 aufweist, der am vorderen Ende 3 in eine Öffnung 3.1 mündet. Der Versorgungskanal 14 ist mit der Wand 10 durch mehrere rippenförmige Längstrennwände 22, 23, 24 und 25 verbunden, die im Behandlungskatheter 1 beim vorliegenden Ausführungsbeispiel vier voneinander unabhängige Kanäle 26, 27, 28 und 29 bilden.

Auf dem Behandlungskatheter 1 sind zwei ausdehnbare Ballons 4.1 und 4.2 aufgebracht, die voneinander im Abstand eines Bereiches 30 zur Behandlung einer Verengung, insbesondere einer Stenose 9, innerhalb eines Gefäßes 2, beispielsweise einer Arterie, angeordnet sind. Der vor dem Bereich 30 vorhandene Ballon 4.1 ist über eine Öffnung 31 in der Wand 6 des Katheters 1 mit dem Kanal 27 und der hinter dem Bereich 30 vorhandene Ballon 4.2 ist über eine Öffnung 32 der Wand 6 des Katheters 1 mit dem Kanal 29 verbunden. Durch den Druck eines in den Kanälen 27 und 29 vorhandenen Mediums, beispielsweise Gas, Wasser oder eine Kochsalzlösung, werden die Ballons 4.1 und 4.2 so weit aufgedehnt, daß sie von innen dichtend an der Gefäßinnenwand 33 beiderseits der Verengung 9 anliegen.

Im Kanal 26 ist eine Lichtleitfaser 11 vorgesehen, in die am nicht dargestellten äußeren Ende dieser Lichtleitfaser 11 Laserlicht eingestrahlt werden kann. Die Lichtleitfaser 11 endet im Katheter 1 im Bereich 30. Ihr Endbereich 21 ist so geführt, daß außerhalb eingestrahltes Laserlicht von innen durch die Wand 10 in den Raum des Bereiches 30 gestrahlt werden kann. Damit ein durch die Öffnung 34 in den Bereich 30 eingebrachtes Fluid vom Laserlicht mit hohem Wirkungsgrad erhitzt werden kann, besteht dieses, wie bereits oben beschrieben, aus einem Material, das die Wellenlänge des verwendeten Laserlichts, beispielsweise eines $CO_2$-Lasers oder eines Neodym-Yttrium-Aluminium-Granat-Lasers (Nd:YAG-Laser) gut absorbiert.

Die Wand des Katheters 1 besitzt im Kanal 26 zwischen den Ballons 4.1 und 4.2 die erwähnte Öffnung 34 und im Kanal 28 eine Öffnung 35. Hierdurch kann der Behandlungskatheter, wie eingangs geschildert, mit dem gewünschten Behandlungsmedium gefüllt, behandelt und das Behandlungsgut anschließend abgesaugt werden.

Falls erforderlich, können zwei oder mehr Lichtleitfasern in einem oder mehreren Kanälen vorgesehen sein. Gegebenenfalls kann es zweckmäßig sein, einen oder mehrere Lichtleitfasern, eventuell zusätzlich, außen am Behandlungskatheter 1, vorzugsweise in wenigstens einer Längsnut 15 anzuordnen (Figur 11).

In Figur 12 ist eine spezielle Anordnung des Endbereiches 21 der in einer Nut 15 des Behandlungskatheters 1 liegenden Lichtleitfaser 11 dargestellt. Hierbei ist der Endbereich 21 am Ende der Nut 15 in einem Winkel $\alpha$ = 3° bis etwa 15° nach außen abgewinkelt. Dadurch wird eine günstige Lichtabstrahlung vom Endbereich 21 in das im Bereich 5 befindliche Fluid erreicht.

Gemäß einer vorteilhaften weiteren Ausgestaltung der Erfindung können die Lichtleitfasern 11 oder wenigstens eine der Lichtleitfasern 11 und ein Lichtverteilelement 100 derart ausgebildet und angeordnet sein, daß die Lichtleitfaser(n) 11 axial, d.h. in Richtung der Längsachse 16, siehe Fig. 13 und 14) des Behandlungskatheters 1 in dieses einstrahlt. Hierzu ist gemäß den Figuren 13 bis 15 das Lichtverteilelement 100 mit einem in das Lumen oder in einen der Kanäle 26 bis 29 hineinragenden Abschnitt 18 versehen. Dieser Abschnitt 18 besitzt auf der der Lichtleitfaser 11 zugewandten Stirnseite 19 eine Aussparung 20 in Form eines axial, d.h. in Richtung der Längsachse 16 des Behandlungskatheters 1 verlaufenden Sackloches. In dieses Sackloch ragt der abstrahlende Endbereich 21 der Lichtleitfaser 11 hinein. Der Endbereich 21 kann in der Aussparung 20, beispielsweise durch Klebung, befestigt sein. Je Aussparung 20 können mehrere Lichtleitfasern 11 vorgesehen sein. Auch können mehrere Aussparungen 20 vorhanden sein, in die jeweils wenigstens eine Lichtleitfaser 11 hineinragt.

Bei allen Ausführungsbeispielen der Figuren 13 bis 15 kann das Lichtverteilelement 100 an seiner Oberfläche mit Rippen oder mit anderen dessen Oberfläche vergrößernden, lichtstreuenden Mitteln versehen sein.

Das Lichtverteilelement 100 kann vollkommen innerhalb des Lumens des Katheters 1, oder, wie in den Figuren 5, 6, 7, 13, 14 und 15 dargestellt, die Katheterwand 6 bzw. 10 durchdringend und eventuell umgreifend oder ganz außerhalb der Katheterwand 6 bzw. 10 angebracht sein.

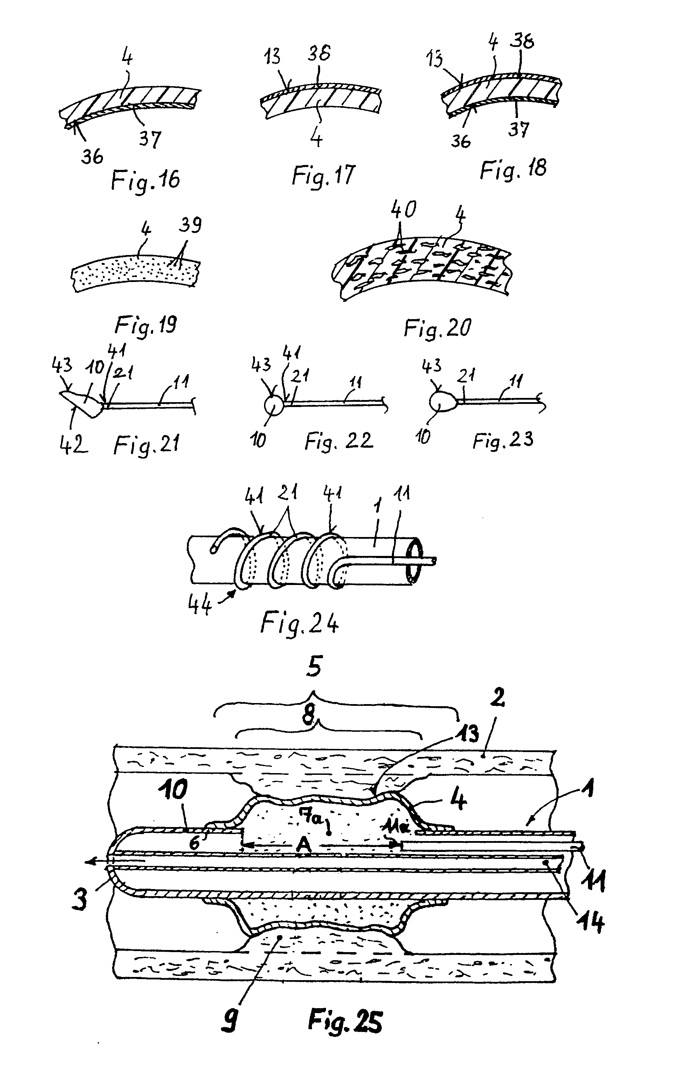

Gemäß einer vorteilhaften anderen Weiterbildung der Erfindung kann die Ballonwand des Ballons 4 lichtreflektierend ausgestaltet sein, damit kein oder möglichst wenig Laserlicht direkt an das Gewebe abgestrahlt werden kann. So kann beispielsweise die Ballonwand des Ballons 4 gemäß Figur 16 auf der Innenseite 36 mit einer Reflexionsschicht 37, oder gemäß Figur 17 auf der Außenseite 13 mit einer Reflexionsschicht 38, oder gemäß Figur 18 sowohl auf der Innenseite 36 als auch auf der Außenseite 13 mit je einer Reflexionsschicht 37 bzw. 38 versehen sein. Als Reflexionsschichten dienen vorzugsweise Metallschichten, die z.B. chemisch abgeschieden oder in sonst geeigneter Weise, z.B. durch Vakuumbedampfung, Sputtern, Shoopen oder dgl. aufgebracht sind. Als Metalle eignen sich vor allem Silber, Aluminium, Zinn, Zink, Chrom, Nickel, Eisen als reine Metalle oder als Mischungen oder Legierungen wenigstens zwei dieser Metall, gegebenenfalls noch mit einem Zusatz von Kupfer.

Anstelle der Reflexionsschichten 37, 38 oder zusätzlich zu diesen, kann auch das elastisch dehnbare Material des Ballons 4 selbst lichtreflektierend ausgebildet sein, z.B. kann es gemäß Figur 19 in feinverteilter Form, insbesondere im Ballonmaterial dispergiert, Partikel 39 enthalten, die ein gutes Reflexionsvermögen und gegebenenfalls ein gutes Streuvermögen für das verwendete Laserlicht aufweisen. Dies können feine Metallpartikel der vorgenannten Metalle sein, z.B. mit einer Partikelgröße von bis zu 0,5 mm, vorzugsweise jedoch kleiner als 10 $\mu$m, insbesondere kleiner als 5 $\mu$m.

Es können jedoch auch Partikel 40 in Form von Schuppen oder Plättchen vorgesehen sein, wie in Figur 20 veranschaulicht ist. Diese legen sich beim Herstellungsprozeß in der Regel parallel zur Wand, so daß gute Reflexionseigenschaften erhalten werden. Als Material für diese Partikel eignet sich Metalle, wie vorstehend beschrieben, deren Legierungen, oder auch Metalloxide, wie Aluminiumoxid, Zirkondioxid, Titandioxid oder dgl..

Gemäß einer weiteren zweckmäßigen Ausgestaltung der Erfindung ist die Oberfläche 41 des Endbereiches 21 der Lichtleitfaser 11 so ausgebildet, daß das Laserlicht über eine größere Fläche abgestrahlt wird als dem lichtleitenden Querschnitt der Lichtleitfaser 11 entspricht. Dadurch ist die abgestrahlte Energiedichte geringer als diejenige in der Lichtleitfaser 11, so daß eine örtliche Überhitzung des aufzuheizenden Fluids und damit beispielsweise eine mögliche Dampfblasenbildung vermieden wird. Dies kann z.B. dadurch erreicht werden, daß der Endbereich 21 das Laserlicht diffus streut, was beispielsweise durch eine Aufrauhung der Oberfläche 41 des Endbereiches 21 der Lichtleitfaser 11, erreicht werden kann. Die Aufrauhung erfolgt beispielsweise durch einen geeigneten Ätzprozeß oder durch Beschichten mit einem lichtstreuenden Material. Auch können, gegebenenfalls zusätzlich, im Endbereich 21 auf das Ende der Lichtleitfaser 11 ein Lichtverteilelement 100 mit einer Prismenfläche 42 aufgebracht, z.B. aus einem Thermoplast angeformt sein, wie in Fig. 21 dargestellt ist. Das Lichtverteilelement 100 kann z.B. gemäß Figur 22 Kugelform oder gemäß Figur 23 Tropfenform besitzen. Beide Lichtverteilelemente 100 können noch zusätzlich eine Prismenfläche aufweisen, auf die der an der Stirnseite des Endbereiches 21 austretende Laserstrahl auftritt und in den Bereich 5 bzw. 30 umgelenkt wird. Auch die Oberfläche 43 des Lichtverteilelements 100 (Figuren 21 bis 23) oder/und das Material des Lichtverteilelements 100 kann bzw. können lichtstreuend ausgebildet sein.

Gemäß einer noch anderen Weiterbildung der Erfindung kann gemäß Figur 24 der Endbereich 21 der Lichtleitfaser 11 spiralförmig ausgeführt und zumindest seine nach außen weisende Oberfläche 41 lichtstreuend ausgeführt sein. Die Spirale 44 umgreift den Katheter 1 oder sie kann auch innerhalb des Katheters 1 angeordnet sein, wobei sie zweckmäßig an dessen Innenwand anliegt.

Nach der in Fig. 25 dargestellten Ausführung verläuft das Ende 11 a der Lichtleitfaser 11 in Längsrichtung des Katheters 1, also axial zu diesem. Im Anschluß an das Ende 11a der Lichtleitfaser 11 weist der Katheter 1 zum Katheterende 3 hin einen Schlitz, eine Nut oder ein Langloch 7 a auf. Dadurch wird im Gegensatz zu den Ausführungsvarianten nach den Figuren 1 und 2 eine axiale Abstrahlung des Laserlichtes über eine größere Absorptionsstrecke A ermöglicht. In entsprechender Weise kann bei der Ausführungsform gemäß Figur 3 durch Verkürzen der Lichtleitfaser 11 die Absorptionsstrecke vergrößert werden.

**Patentansprüche**

1. Behandlungskatheter zur Verringerung oder Beseitigung von Engstellen in Körperflüssigkeit führenden Gefäßen, mit wenigstens einem über ein Lumen oder über einen Kanal durch ein flüssiges Medium aufweitbaren Ballon, sowie mit einer Lichtleitfaser, durch die ein von außen in dieses bzw. in diesen eingeleitetes Laserlicht beim im Gefäß entsprechend positioniertem Katheter im Bereich der Engstelle abstrahlbar und durch die abgestrahlte Lichtenergie das Gewebe der Engstelle erhitzbar, insbesondere denaturierbar ist, dadurch gekennzeichnet, daß wenigstens eine Lichtleitfaser (11) entweder

a) im Bereich (5) innerhalb des bzw. eines Ballons (4) derart ausgebildet ist, daß die einstrahlbare Laserenergie, insbesondere mit gegenüber der im Lichtleiterquerschnitt vorhandenen Energiedichte geringerer Energiedichte, in den Ballon (4) abstrahlbar ist und daß der Ballon (4) mit einem Fluid füllbar und aufweitbar ist, das ein hohes Absorptionsvermögen für die Wellenlänge des verwendeten Laserlichts besitzt und das gegebenenfalls zusätzlich ein hohes Streuvermögen für einstrahlendes Laserlicht der verwendeten Wellenlänge besitzt, oder

b) im Bereich (30) zwischen zwei aufweitbaren Ballons (4.1, 4.2) an der Außenwand (10) des Katheters (1) endet und dort derart ausgebildet ist, daß die einstrahlbare Laserenergie, insbesondere mit gegenüber der im Lichtleiterquerschnitt vorhandenen Energiedichte geringerer Energiedichte, in den Bereich (30) abstrahlbar ist, und daß im Bereich (30) zwischen den beiden Ballons (4.1, 4.2) wenigstens eine Öffnung (34, 35) in der Katheterwand (10) vorgesehen ist, in die ein gesonderter Kanal (26) des Katheters (1) mündet, über die der Bereich (30) mit einem Fluid füllbar oder/und spülbar ist, das ein hohes Absorptionsvermögen für die Wellenlänge des verwendeten Laserlichts besitzt und das gegebenenfalls zusätzlich ein hohes Streuvermögen für einstrahlendes Laserlicht der verwendeten Wellenlänge besitzt.

2. Behandlungskatheter nach Anspruch 1, dadurch gekennzeichnet, daß bei im Bereich (5) innerhalb eines Ballons (4) abstrahlender Lichtleitfaser (11) die Ballonwand für das verwendete Laserlicht gut reflektierend ausgebildet ist.

3. Behandlungskatheter nach Anspruch 2, dadurch gekennzeichnet, daß die Ballonwand innen und/oder außen mit einer Reflexionsschicht (37, 38) versehen ist.

4. Behandlungskatheter nach Anspruch 3, dadurch gekennzeichnet, daß die Reflexionsschicht (37, 38) aus einer Metallschicht besteht.

5. Behandlungskatheter nach Anspruch 4, dadurch gekennzeichnet, daß die Metallschicht aus wenigstens einem der Metalle Eisen, Chrom, Nickel, Aluminium, Silber, Zinn oder Zink oder aus einer Legierung wenigstens zwei dieser Metalle, gegebenenfalls zusätzlich mit Kupfer, besteht.

6. Behandlungskatheter nach einem der Ansprüche 1 bis 5, dadurch gekennzeichnet, daß die Ballonwand aus elastisch dehnbarem Material besteht, in dem gut reflektierende Partikel, vorzugsweise aus Metall, dispergiert sind.

7. Behandlungskatheter nach Anspruch 6, dadurch gekennzeichnet, daß die Partikel die Form dünner Plättchen oder Schuppen besitzen.

8. Behandlungskatheter nach einem der Ansprüche 1 bis 7, dadurch gekennzeichnet, daß das Ende (21) der Lichtleitfaser (11) im Bereich (5) des Ballons (4) bzw. im Bereich (30) zwischen den beiden Ballons (4.1, 4.2), gegebenenfalls an der Außenwand des Katheters (1), in Längsrichtung des bzw. axial zum Katheter(s) (1) oder unter einem Winkel ($\alpha$) von etwa 3° bis zu etwa 15° gegenüber der Längsachse (16) des Katheters (1) geneigt verläuft.

9. Behandlungskatheter nach einem der Ansprüche 1 bis 7, dadurch gekennzeichnet, daß das Ende (21) der Lichtleitfaser (11) im Bereich (5) des Ballons (4) bzw. im Bereich (30) zwischen den beiden Ballons (4.1, 4.2) innen oder außen an der Außenwand des Katheters (1) spiralförmig verläuft.

10. Behandlungskatheter nach Anspruch 8 oder 9, dadurch gekennzeichnet, daß die frei in den Ballon (4) bzw. den Bereich (30) zwischen den beiden Ballons (4.1, 4.2) ragende Oberfläche (41) des Endabschnitts der Lichtleitfaser (11) aufgerauht ist.

11. Behandlungskatheter nach einem der Ansprüche 1 bis 10, dadurch gekennzeichnet, daß auf das stirnseitige Ende (21) der Lichtleitfaser (11) ein prismen-, kugel- oder tropfenförmiges Lichtverteilelement (10) aufgebracht, insbesondere aufgeformt, aufgeklebt oder aufgeschmolzen ist.

12. Behandlungskatheter nach Anspruch 11, dadurch gekennzeichnet, daß das Lichtverteilelement (10) und/oder dessen Oberfläche (43) lichtstreuend ausgebildet ist.

Fig. 1

Fig. 2

Fig. 3

Fig. 4

Fig. 5

Fig. 6

Fig. 7

Fig. 8

Fig. 9

Fig. 12

Fig. 11

**Fig. 13**

**Fig. 14**

**Fig. 10**

**Fig. 15**

Fig. 16

Fig. 17

Fig. 18

Fig. 19

Fig. 20

Fig. 21

Fig. 22

Fig. 23

Fig. 24

Fig. 25